# EUROPEAN PATENT SPECIFICATION

(11) **EP 0 745 382 B1**
(45) Date of publication and mention of the grant of the patent: **12.11.2003**
(21) Application number: 95906533.5
(22) Date of filing: 27.01.1995
(51) Int. Cl.: A61K 9/20, A61K 47/26, A61K 9/00

(54) **INTRAORALLY SOLUBLE COMPRESSED MOLDING AND PROCESS FOR PRODUCING THE SAME**
INTRAORAL LÖSLICHER FORMPRESSLING UND VERFAHREN ZU SEINER HERSTELLUNG
MOULAGE COMPRIME A SOLUBILITE INTRA-ORALE ET SON PROCEDE DE PRODUCTION

(30) Priority: 31.01.1994 JP 1011294; 25.04.1994 JP 8665294
(43) Date of publication of application: 04.12.1996
(73) Proprietor: YAMANOUCHI PHARMACEUTICAL CO. LTD., Tokyo 103 (JP)
(72) Inventor: MIZUMOTO, Takao, Haibara-gun Shizuoka 421-03 (JP); MASUDA, Yoshinori, Shizuoka 425 (JP); FUKUI, Muneo, Shizuoka 426 (JP)
(74) Representative: Geering, Keith Edwin
(86) International application number: JP9500102
(87) International publication number: WO95020380

(56) References cited:
- EP-A- 0 409 279
- WO-A-93/11752
- DE-A- 1 617 638
- DE-A- 2 658 282
- JP-A- 5 271 054
- JP-A- 5 310 558
- US-A- 3 619 292
- US-A- 3 627 583
- US-A- 3 873 694
- US-A- 4 698 101
- US-A- 5 204 115

## Description

The present invention relates to intrabuccally dissolving compressed moldings which quickly disintegrate and dissolve in the buccal cavity and have an adequate hardness and to production processes thereof. Particularly, the present invention relates to intrabuccally dissolving compressed moldings which are useful in the pharmaceutical field.

The term "intrabuccally dissolving compressed moldings" as used herein means compressed moldings which show practically sufficient disintegration and dissolution by saliva by merely keeping in the mouth without holding water in the buccal cavity and which have adequate hardness. The term "practically sufficient disintegration and dissolution" as used herein means that the moldings disintegrate or dissolve in the buccal cavity within approximately 1 to 120 seconds, preferably within approximately 1 to 60 seconds, and more preferably within approximately 1 to 40 seconds. The term "adequate hardness" means that the moldings have a sufficient hardness so that the molding do not break during the production steps and distribution stages.

Dosage forms in which easy swallowing by patients is taken into consideration are scarce in spite of the existence of various known dosage forms of pharmaceutical preparations for oral administration use. Accordingly, great concern has been directed toward the development of a dosage form which can easily be handled, especially by the aged or children having difficulty in swallowing preparations.

For example, in the case of tablets and capsules frequently used as oral preparations, many aged or children having weak swallowing power are unwilling to take these solid preparations complaining that the drug is difficult to swallow or stops in the pharynx or gullet. Chewable tablets are not suitable for the aged or children having weak chewing power.

In the case of powders and granules, they are difficult to swallow because of their aptness to remain in the buccal cavity and, therefore, to cause a unpleasant feeling in the mouth. In some cases, the aged will be choked with powders or feel a pain or unpleasantness due to granules getting between false teeth. In addition, powders and granules have to be used after tearing each package, but the aged or children often have difficulty in tearing the package or spill a portion of its contents.

To take these oral preparations, it is necessary to use water, and the aged or children especially require a large volume of water in many cases because of the swallowing difficulty. However, there is a situation that it is necessary to drink water moderately, especially before retiring to bed because of the urination problem at night. In addition, in the case of patients who have to take oral preparations constantly while continuing daily life, water can hardly be obtained in certain cases depending on circumstances, thus sometimes entailing decline in compliance.

Syrups and the like are regarded as desirable dosage forms for the aged or children, but the aged or children who have difficulty in measuring the necessary volume cannot be expected to use such preparations in the correct dose. In addition, since there are many aged patients who can hardly take liquid preparations by mouth by themselves, such dosage forms cannot always be regarded as suitable dosage forms for the aged and children unless the patient can ask a nurse for a helping hand.

Taking such circumstances into consideration, attempts have been made to develop the following intrabuccally dissolving preparations suitable for the aged and children. However, they are not practically sufficient because of disadvantages such as (1) complex production steps and new plant and equipment investment required for the production of such preparations, (2) limitation in the application of the active ingredients and (3) difficulty in handling the preparations due to their inadequate hardness accompanied by the quick disintegration and dissolution in the buccal cavity.

When the forthcoming social condition of advanced age is taken into consideration, development of a practical preparation which can be used easily, especially by the aged, seems to be an immediate need, because the morbidity rate of chronic diseases increases with advance in age and patients of advanced age have a tendency to take drugs for a long period of time. Also, in order to keep the quality of life, it is desirable to develop a preparation which can be easily swallowed and handled in accordance with the ability and life condition of each patient.

An examined Japanese patent publication No. 58-24410 discloses a process for the production of porous tablets having an excellent disintegration property, which comprises mixing contents of the tablet with a solvent that is inert to the contents of the tablet and that freezes at a temperature in the range of from -30°C to +25°C, said solvent being used in an amount of from 5 to 80% by weight based on the total mixture, solidifying the mixture by putting the mixture in an inert cooling medium, compressing the solidified mixture at a temperature lower than the freezing point of the solvent to make the mixture into tablets, and then conducting volatilization of the solvent by means of freeze drying, spontaneous drying or the like.

An unexamined published Japanese patent application No. 3-86837 discloses an easily dissolvable carrier material having a sufficient strength which is obtained by allowing a composition comprising a water-soluble hydrous gel or a foam substance to contact with an anhydrous organic liquid desiccating agent such as anhydrous ethanol at a temperature of about 0°C or lower until all of the water content is substantially removed from the composition.

Each of these production processes, however, requires complex production steps and additional manufacturing facilities such as a freeze dryer and the like, thus entailing high production cost.

An unexamined published Japanese patent application No. 2-32014 discloses a solid preparation in the form of tablets by wet-production which are suitable for oral administration. However, since these tablets are obtained by preparing a wet mass using ethanol/water or water alone and drying the mass in a mold, the process to produce these tablets has poor productivity in comparison with the common production processes.

An unexamined published Japanese patent application No. 61-15830 discloses an antacid composition having a porous ultra-fine crystal structure, which contains an antacid agent, a sweet material for confectionery use and confectionery base containing a plasticizer. An unexamined published Japanese patent application No. 3-209336 discloses a pharmaceutical composition which is obtained by uniformly dispersing particles of at least one pharmaceutically active compound in the crystal matrices of a crystalline sugar alcohol derived from at least one monosaccharide or polysaccharide. Each of these production processes, however, has a disadvantage in that the application of active ingredients is limited in view of the heat-stability because of the step to melt sugar components at 100°C or higher.

In addition, though an intrabuccally dissolving pharmaceutical preparation is now commercially available from R.P Scherer Company under the trade name "Zydis", it is highly costly because it requires an additional manufacturing facility such as a freeze dryer or the like due to its production by freeze drying and it requires a prolonged period of time for the production. Also, since the pharmaceutical preparation obtained by freeze drying has a weak strength, it requires special cautions for its handling and, therefore, is not satisfactory for the use by the aged. For example, unlike the case of usual tablets, this preparation cannot easily be taken from a package by pressing the package (PTP: press through package).

The above-mentioned intrabuccally dissolving pharmaceutical preparation obtained by freeze drying (to be referred to as the "freeze-dried preparation" hereinafter) is excellent especially in disintegration and dissolution, but is not satisfactory in terms of its storage life because it does not have a sufficient hardness for keeping its dosage forms during the production steps and distribution stages.

In addition to the conventional freeze drying method, other intrabuccally dissolving pharmaceutical preparations produced by tabletting have been reported.

An unexamined published Japanese patent application No. 5-271054 discloses that intrabuccally dissolving tablets having an adequate strength and a porous structure which quickly disintegrate and dissolve in the buccal cavity can be obtained by preparing a mixture of an active ingredient, a saccharide and water in a sufficient amount to wet the surface of the saccharide granules, tabletting the mixture into tablets and drying the tablets.

Each of the above-mentioned intrabuccally dissolving pharmaceutical preparations obtained by tabletting (to be referred to as the "tabletted preparation" hereinafter) does not require the production steps for obtaining freeze-dried preparations and is satisfactory in terms of the storage life due to its sufficient hardness for keeping its dosage forms during the distribution stages. However, since the tabletted preparation is produced by simply subjecting a mixture or a blend to tabletting, there is still great room for the improvement of its quick disintegration and dissolution in the buccal cavity which are the characteristics of intrabuccally dissolving pharmaceutical preparations.

In addition, the following documents focused on the moldability of the saccharide and on the direct tabletting. An unexamined published Japanese patent application No. 5-310558 discloses that, when mannitol or lactose having low binding property and poor moldability is blended with sorbitol granules having a bulk density of less than 60 g/100 ml, amounts of other additives having high moldability such as cellulose compounds, acrylic acid compounds, gelatin and the like can be reduced and solid pharmaceutical compositions having excellent disintegration property can be obtained. Similarly, an unexamined published Japanese patent application No. 59-118058 and DE-A-1617638 disclose a preparation in which sorbitol having a particular bulk density is used. These documents may suggest that sorbitol having a particular bulk density can work as a binding agent when the direct tabletting is carried out. However, the inventions of these documents relate to an additive and a production process for obtaining tablets having an improved hardness under the usual tabletting pressure for making tablets, and their object is a production of an additive for the direct tabletting.

According to an unexamined published Japanese patent application No. 5-170669, moldability of lactose is improved by adding a sugar alcohol to a lactose having a high β-lactose content and drying the aqueous solution thereof by means of roller drying. However, since special saccharides are required, these processes are complex and expensive and, therefore, is not practical.

U.S. Patent No. 4,698,101 discloses a pharmaceutical adjuvant based on fructose which is obtained by granulating fructose with an aqueous maltose solution and which can be subjected to direct tabletting.

An unexamined published Japanese patent application No. 4-505918 based on a PCT application discloses a pharmaceutical adjuvant based on fructose which is obtained by granulating fructose with an aqueous polyol solution containing sorbitol, maltitol, lactitol, xylitol, mannitol, isomaltol, or a mixture thereof and which can be subjected to direct compression.

Although fructose is used as a core for the granulation, these documents relate to conventional tablets, not to intrabuccally dissolving tablets. In addition, there is a problem that, when handled usually, the granules absorb moisture due to the high hygroscopicity of fructose and, as a result, sufficient fluidity cannot be obtained, resulting in a tendency to hinder the tabletting.

US-A-3,873,694 discloses a direct tabletting vehicle prepared by mixing a crystalline sugar with maltodextrin and granulating the mixture with molding maltodextrin. US-A-4,698,101 discloses a direct tabletting filler prepared by granulating crystalline fructose with a maltose-containing syrup. US-A-5,204,115 discloses xylitol granules which have good fluidity and can be directly tabletted, prepared by granulating xylitol with a polymerized reducing sugar or the like. US-A-3,627,583 discloses a method for preparing tablets, which includes mixing an aggregate granulated from a fine particulate saccharide such as a mono-, di- or tri-saccharide and a polyhydroxy compound such as a polyhydric alcohol or a saccharide with an active ingredient. US-A-3,619,292 discloses a method for preparing tablets which comprises mixing a powdery aggregate obtained by spray-drying a suspension (saturated solution) of a starch hydrolysate (containing dextrose and oligosaccharides as impurities) with a necessary filler (also containing a drug and the like) and then compressing the mixture. These inventions are to improve fluidity of granules and improve hardness of usual tablets or chewable tablets. The prior products may have strong hardness, but the prior proposals do not achieve a combination of good hardness with quick disintegration and dissolution in the buccal cavity.

DE-A-2658282 discloses compression molding a composition formed by dry mixing xylitol with polyol such as sorbitol or mannitol; it does not suggest coating or granulating the xylitol.

The present invention provides an intrabuccally dissolving compressed molding which shows disintegration and dissolution in the buccal cavity within 1 to 120 seconds and which comprises (a) granules comprising first saccharide coated and/or granulated with second saccharide as a binder, and (b) optionally an active ingredient, said first saccharide being at least one saccharide selected from lactose, mannitol, glucose, sucrose, and xylitol and showing a hardness of 0 to 2 kg when 150 mg of the saccharide is tabletted using a punch of 8 mm diameter under a pressure of 10 to 50 kg/cm², and said second saccharide being at least one saccharide selected from maltose, maltitol, sorbitol and lactosucrose and showing a hardness of 2 kg or more when 150 mg of the saccharide is tabletted using a punch of 8 mm diameter under a pressure of 10 to 50 kg/cm².

It also provides a process for producing a molding according to the invention which comprises coating and/or granulatingsaid first saccharide using said second saccharide as a binder, and subjecting the resulting granules to compression molding.

The present invention can provide (1) an intrabuccally dissolving compressed molding which shows quick disintegration and dissolution in the buccal cavity and has a hardness sufficient to avoid breakage, (2) a process for the production of the intrabuccally dissolving compressed molding by generally used production steps, (3) an intrabuccally dissolving compressed molding which can easily be taken without water and a process for the production thereof and (4) a useful intrabuccally dissolving compressed molding which is excellent in its industrial productivity and has uniformity of active ingredient content and constancy of dosage forms.

In general, products obtained by compression such as tabletting (compressed moldings such as tablets) have had adequate hardness as moldings, However, they have been produced without taking quick disintegration and dissolution in the buccal cavity into consideration, because their object was to effect absorption of active ingredients by disintegration and dissolution of the orally administered moldings in the digestive tract. Accordingly, their disintegration and dissolution in the buccal cavity were not sufficient for the purposes of present invention.

In order to solve the aforementioned problems, a raw material for the construction of intrabuccally dissolving compressed moldings should simultaneously have the following characteristics - quick dissolution rate in the buccal cavity and high moldability to give an adequate hardness, when produced by compression molding such as tabletting.

The inventors of the present invention have examined, as the raw material for intrabuccal compressed moldings, saccharides previously used generally as an additive such as a vehicle.

The intrabuccal dissolution time and the hardness of tablets, obtained by subjecting various saccharides commonly used as additives (such as a vehicle for a pharmaceutical preparation) to tabletting under a pressure of 10 to 50 kg/cm² were measured.

This did not reveal a raw material simultaneously satisfying the aforementioned two characteristics, but instead the unexpected. new finding that saccharides are divided into two groups, those showing quick dissolution rate in the buccal cavity when made into tablets and those having high moldability to give an adequate hardness.

However, when a low moldability saccharide or a high moldability saccharide was used alone in the compression molding, adequate hardness and quick disintegration and dissolution in the buccal cavity were not simultaneously obtained.

Low moldability saccharide was poor in moldability, but showed a markedly high dissolution time of not more than about 15 seconds in the buccal cavity when made into tablets. However, a sufficient hardness was not obtained. For example, when 150 mg of low moldability saccharide is made into a tablet using a punch of 8 mm diameter (φ) under a pressure of 50 kg/cm², a sufficient hardness of the tablet was not obtained.

High moldability saccharide was excellent in moldability as a matter of course, but its disintegration property in the buccal cavity was inferior to that of low moldability saccharide.

For example, when 150 mg of high moldability saccharide is made into a tablet using a punch of 8 mm diameter (φ) under a pressure of 50 kg/cm², quick disintegration and dissolution in the buccal cavity were not achieved though a sufficient hardness of the tablet was obtained.

Moreover, quick disintegration and dissolution in the buccal cavity were not obtained when a low moldability saccharide and a high moldability saccharide were simply mixed (physical mixture) and tabletted. For example, when 189 g of lactose, 10 g of maltitol and 1 g of magnesium stearate were mixed and the mixture was tabletted into tablets each weighing 300 mg by a rotary tabletting machine with a punch of 10 mm radius (R 10 mm) and φ10 mm under a pressure of 441 kg/cm², quick disintegration and dissolution in the buccal cavity were not obtained.

The present inventors have conducted extensive studies on the combination of low moldability saccharides having high dissolution with high moldability saccharides, such as on their blending ratio and blending method, with the aim of finding a method which can simultaneously satisfy the two characteristics of improved moldability of a low moldability saccharide to obtain an adequate hardness after compression molding while keeping its quick dissolution rate. As a result, a raw material of interest was obtained by improving the defects of the saccharides having inferior moldability but excellent disintegration and dissolution, namely low moldability saccharides, and defects of the high moldability saccharides; this was achieved by subjecting a low moldability saccharide to granulation with a high moldability saccharide as a binder. Compressed moldings obtained from this raw material, by a generally used compression molding step such as tabletting, showed an adequate hardness and quick disintegration and dissolution when kept in the mouth.

In other words, the present inventors have conducted extensive studies on intrabuccally dissolving compressed moldings with the aim of solving the aforementioned problems and found that, when a raw material obtained by granulating a low moldability saccharide showing quick disintegration and dissolution with a high moldability saccharide as binder is subjected to conventional molding, the resulting moldings show quick disintegration and dissolution in the buccal cavity by merely keeping in the mouth and have adequate hardness, so that the dosage forms do not break during the production steps and distribution stages. The present invention was accomplished on the basis of this finding.

In addition, the intrabuccally dissolving compressed moldings of the present invention are completely different from the conventional intrabuccally dissolving preparations in terms of their composition and shape, because a high moldability saccharide was used as a binding agent in the granulation step instead of the commonly used water-soluble polymer binders such as hydroxypropylcellulose (HPC) and hydroxypropylmethylcellulose (HPMC).

Also, the present invention was accomplished on the basis of another finding that a low moldability saccharide granulated with a high moldability saccharide binder is a raw material useful for providing intrabuccally dissolving compressed moldings, preferably intrabuccally dissolving tablets.

The term a "low moldability saccharide" as used herein as one of the components of the present invention means a saccharide which shows a hardness of 0 to 2 kg when 150 mg of the saccharide is made into a tablet using a punch of 8 mm diameter (φ) under a pressure of 10 to 50 kg/cm². Examples of such saccharides include the lactose, mannitol, glucose, sucrose and xylitol which are used in the invention and of which lactose and mannitol are preferred.

These saccharides may be used alone, or as a mixture of two or more, as said first saccharide in the invention.

The term a "high moldability saccharide" as used herein means a saccharide which shows a hardness of 2 kg or more when 150 mg of the saccharide is made into a tablet using a punch of 8 mm diameter (φ) under a pressure of 10 to 50 kg/cm². Examples of such saccharides include the maltose, maltitol, sorbitol, and lactosucrose which are used in the invention and of which maltose and maltitol are preferred. A suitable lactosucrose powder is Nyuka Oligo LS-55P (a product name) manufactured by Hayashibara Shoji Co., Ltd.

These saccharides may be used alone or as a mixture of two or more, as a said second saccharide in the invention.

The intrabuccally dissolving compressed molding of the present invention uses a low moldability saccharide as its main component, with a blending ratio of high moldability saccharide to low moldability saccharide being from 2 to 20%, preferably from 5 to 10%.

If the blending ratio is smaller than 2%, adequate hardness of the tablets cannot be obtained, resulting in easy breakage of the tablets during their storage or transportation or when they are taken out from packages. If the blending ratio is larger than 20%, the hardness of the tablets becomes excessive and the desired quick disintegration and dissolution in the buccal cavity cannot be obtained. More preferably, the blending ratio may be in the range of from 5 to 10% from the view point of effective granulation on industrialisation of the process.

More preferably, granules obtained by granulating lactose and/or mannitol (low moldability) with, as binder, maltose or maltitol (high moldability) in an amount of from 5 to 7.5% by weight based on the total weight of the intrabuccally dissolving compressed molding are used for the present invention.

The active ingredient may be mixed by (1) mixing it with said first saccharide or (2) mixing it with granules obtained by coating and/or granulating said first saccharide with said second saccharide as a binder. Alternatively active ingredient may be mixed by (3) mixing granules obtained by coating and/or granulating said first saccharide with a said second saccharide as a binder and granules obtained by coating and/or granulating an active ingredient with a said second saccharide as a binder, (4) coating and/or granulating said first saccharide with both an active ingredient and said second saccharide binder in any order, (5) coating said first saccharide (central core) with a said second saccharide as a binder (first layer), then with an active ingredient (second layer), and coating the product with a said second saccharide as a binder, or (6) coating said first saccharide with an active ingredient and coating and/or granulating the coated product with said second saccharide as a binder. The second saccharide is preferably used in an amount of 5 to 7.5% by weight based on the total weight of the intrabuccally dissolving compressed molding, e.g., the total weight of saccharide or the total weight of saccharide and active ingredient.

The particle size distribution and the particle diameter of the granules may vary as long as fluidity is maintained, and the usual particle size distribution for tabletting may be employed. For example, the particle size may be 1000 - 10 µm.

Active ingredients may be included in the preparation of the present invention, with their preferred examples including drugs for patients having difficulty in swallowing tablets, the aged and children, drugs for patients who require drug-taking without water while spending daily life, preparations for patients whose water drinking is limited and drugs for use in potions.

Illustrative examples of drugs having high utility values include:
Antacids such as sodium hydrogencarbonate, dried aluminum hydroxide gel, calcium carbonate, magnesium hydroxide, magnesium alminate silicate, synthetic aluminum silicate, synthetic hydrotalcite, magnesium aluminum hydroxide, aluminum hydroxide gel, coprecipitated product of aluminum hydroxide and sodium hydrogencarbonate, mixed dried gel of aluminum hydroxide and magnesium carbonate, coprecipitated product of aluminum hydroxide, magnesium carbonate and calcium carbonate, aluminum magnesium metasilicate, aluminum bismagnesium bismuth silicate, coprecipitated product of magnesium hydroxide and aluminum potassium sulfate, powdered oyster shell, aminoacetic acid and scopolia extract,
serotonin 5HT₃ receptor antagonists such as (R)-5-[(1-methyl-3-indolyl)carbonyl]-4,5,6,7-tetrahydro-1H-benzimidazole hydrochloride and salts thereof, ondansetron and granisetron,
non-steroidal anti-inflammatory drugs such as indometacin, ibuprofen, ibufenac, alclofenac, diclofenac, mefenamic acid, flurbiprofen, flufenamic acid, ketoprofen, phenylbutazone and methyl salicylate,
   steroidal anti-infammatory drugs such as cortisone, hydrocortisone, prednisolone, dexamethasone, betamethasone dipropionate, betamethasone valerate, prednisolone, triamcinolone and fluocinolone acetonide,
   diuretic drugs such as bendroflumethiazide, polythiazide, methyclothiazide, trichlormethiazide, cyclopenthiazide, pentylhydrochlorothiazide, hydrochlorothiazide and bumetanide,
antipsychotic drugs such as emonapride, diazepam, nitrazepam, flunitrazepam, lorazepam, prazepam, fludiazepam, clonazepam, chlorpromazine, reserpine, clofluperol, trifluperidol, haloperidol, moperone, bromperidol and etizolam,
hypnotic drugs such as barbital, thiopental, phenobarbital, cyclobarbital, lormetazepam, triazolam and alprazolam, like,
   antiepileptic drugs such as ethosuximide, sodium valproate, acetazolamide and meprobamate,
   antiparkinsonism drugs such as chlorzoxazone and levodopa,
antiemetic drugs such as metoclopramide and metoclopramide hydrochoride,
   hormone drugs such as insulin, testosterone, methyltestosterone, progesterone and estradiol,
   analgesic drugs such as morphine, aspirin, codeine, acetanilide, aminopyrine and loxoprofen,
   sulfa drugs such as sulfamine, sulfamonomethoxine and sulfamethizole,
   coronary vasodilators such as nitroglycerin, isosorbide dinitrate, pentaerythrityl tetranitrate, propatylnitrate, dipyridamole and papaverine HCl,
   H₂ receptor antagonists such as famotidine, cimetidine, ranitidine HCl and roxatidine acetate HCl,
   antiarrhythmic drugs such as ajimalin, pindolol, propranolol, quinidine, amrinone and milrinone,
   cardiotonic drugs such as caffeine, digoxin and digitoxin, calcium antagonists such as nicardipine HCl, diltiazem HCl, nivadipine, nifedipine, nitrendipine, nisoldipine, nimodipine and niludipine,
   antihistaminic drugs such as diphenhydramine HCl, carbinoxamine, diphenylpyrallin, phenbenzamine, chlorpheniramine maleate, brompheniramine maleate, diphenylimidazol and clemizole,
   antibiotics such as tetracycline, oxytetracycline, metacycline, doxycycline, minocycline, chloramphenicols, erythromycins, lincomycin, penicillin G, clindamycin, kanamycin, chloramphenicol, fradiomycin, streptomycin and gentamicin,
   antitumor drugs such as 5-fluorouracil, uracil, cytarabine, floxuridine, busulfan, actinomycin, bleomycin and mitomycin,
antidiabetic drugs such as glibenclamide and epalrestat,
gout treating drugs such as allopurinol, colchicine and benzbromarone,
   antiallergic drugs such as ketotifen fumarate, sodium cromoglicate and amlexanox,
   antihypertensive drugs such as clonidine, atenolol, doxazosin, bisoprolol, cilazapril, lisinopril, nilvadipine, manidipine, isosorbide dinitrate, diltiazem, nicorandil, guanethidine sulfate, amosulalol HCl, alacepril, delapril HCl and enalapril maleate,
central nervous system acting drugs such as indeloxazine HCl, tiapride HCl and bifemelane HCl,
   potassium channel activator such as YM934 (2-(3,4-dihydro-2,2-dimethyl-6-nitro-2H-1,4-benzoxazin-4-yl)pyridine N-oxide),
   skeletal muscle relaxants such as sodium dantrolene,
antispasmodic drugs such as eperisone HCl, tizanidine HCl, butylscopolamine and atropine methylbromide, antihyperlipemic drugs such as simvastatin and prevastatin sodium,
   bronchodilators such as formoterol fumarate, salbutamol sulfate and procaterol HCl,
   α-adrenergic receptor blockers such as tamsulosin hydrochloride and prazosin,
   blood sugar lowering drugs,
   oral contraceptives,
   analgesic/anti-infammatory drugs such as loxoprofen,
digestive tract motility improving drugs such as domperidone and cisapride,
   antigastritis and antigastric ulcer drugs such as teprenone,
osteoporosis treating drugs such as alfacalcidol,
prostatonegaly treating drugs such as chlormadinone acetate,
expectorants such as ambroxol,
   allergic rhinitis treating drugs such as oxatomide and ketotifen,
   asthma treating drugs such as azelastine, procaterol and terfenadine, and
   animal drugs having antipyretic/analgesic/anti-inflammatory activities and peptic antiulcer activities and animal organ drugs for treating reproductive organs.

In addition, since the intrabuccally dissolving compressed molding of the present invention is taken with disintegration and dissolution in a buccal cavity, it can be applied to the cases in which an active ingredient is absorbed in the buccal cavity as the occasion demands. In this regard, the following peptides can be exemplified in addition to the aforementioned active ingredients.

As representative peptides, various polypeptides, proteins and derivative thereof, which are liable to be degraded in the upper digestive tract but are absorbed in the lower digestive tract and show physiological effects can be used effectively as the active ingredient for the present invention. Examples of such peptides include insulin, calcitonin, angiotensin, vasopressin, desmopressin, LH-RH (luteinizing hormone-releasing hormone), somatostatin, glucagon, oxytocin, gastrin, ciclosporin, somatomedin, secretin, h-ANP (human atrial natriuretic peptide), ACTH (adrenocorticotropic hormone), MSH (melanophore-stimulating hormone), β-endorphin, muramyl dipeptide, enkephalin, neurotensin, bombesin, VIP (vasoactive intestinal polypeptide), CCK-8 (cholecystokinin-8), PTH (parathyroid hormone), CGRP (calcitonin gene related-peptide), TRH (thyrotropin-releasing hormone), endothelin, hGH (human growth hormone), and cytokines such as interleukin, interferon, colony-stimulating factor, and tumor necrosis factor, as well as derivatives of these peptides.

The aforementioned peptides and proteins include their pharmacologically active derivatives and homologues in addition to the naturally-derived ones. For example, the calcitonin which can be used in the present invention includes its analogues such as [Asul,7]-eel calcitonin (elcatonin) in addition to the naturally existing products such as salmon calcitonin, human calcitonin, porcine calcitonin, eel calcitonin, and fowl calcitonin. As to insulin, human insulin, porcine insulin and bovine insulin and, in addition, their homologues such as genetic recombinants, are included.

Preferable active ingredients used in the present invention are famotidine, tamsulosin hydrochloride, and YM934.

As active ingredients, not only pharmaceutical drugs but also various other substances can be used in the present invention making use of its characteristic nature including, for example, diagnostic drugs such as a contrast medium, healthy food, physiologically functional food and buccals such as a bad breath eliminating drug and dental plaque disclosing agent.

Preferably, the active ingredient may be used in an amount of 50% (w/w) or less, preferably 20% (w/w) or less, based on the total solid components, though it varies depending on the nature of each active ingredient to be used.

The raw material of the intrabuccally dissolving compressed moldings of the present invention is obtained by coating and/or granulating said low moldability first saccharide with said high moldability second saccharide as binder, making possible production of intrabuccally dissolving compressed moldings which quickly disintegrate and dissolve in the buccal cavity. In consequence, when the compressed moldings of the present invention are produced, the desired objects can be achieved by adding an active ingredient of interest at any step of the production process. The active ingredient may be present in any moiety of the intrabuccally dissolving compressed moldings of the present invention.

Illustrative examples of the active ingredient-containing raw materials include a raw material which comprises (I) granules obtained by coating and/or granulating an active ingredient and said first saccharide using said second saccharide as a binder, (II) an active ingredient and granules obtaining by coating and/or granulating said first saccharide using said second saccharide as a binder, (III) granules obtained by coating and/or granulating said first saccharide using a said second saccharide as a binder and granules obtained by coating and/or granulating an active ingredient using a said second saccharide as a binder, or (IV) granules obtained by coating and/or granulating said first saccharide with both an active ingredient and said second saccharide binder in any order; or which is obtained (V) by coating said first saccharide (core) with a said second saccharide as a binder (first layer), coating the product with an active ingredient (second layer) and then coating this product with a said second saccharide as a binder (third layer), or (VI) by coating said first saccharide with an active ingredient and coating and/or granulating the coated product using said second saccharide as a binder.

The active ingredient may be added to any part of the composition or at any product step.

A particularly preferred embodiment is the above construction (V) when the dose of active ingredient is extremely small and uniformity of active ingredient content is hard to obtain.. When the active ingredient has low moldability, the above construction (I) or (III) is preferable.

Each of the intrabuccally dissolving compressed moldings of the present invention can contain an active ingredient, said low moldability first saccharide and said high moldability second saccharide and is obtained by subjecting granules finally granulated with said high moldability saccharide binder to compression molding.

In general, active ingredients which do not generate unpleasant taste at the time of their dissolution are preferably used. When a component which generates unpleasant taste is used, it is preferable to employ a proper masking treatment (e.g., WO 92/09275).

In addition, when an active ingredient is desired to be made into a sustained release form, it is preferable to carry out an appropriate conventional sustained release treatment (e.g., Canadian Patent No. 2,038,400-0) so that release of the active ingredient from the resulting granules can be controlled.

The solid preparation of the present invention has sufficient strength for handling (particularly, for handling relating to preparation) and can therefore be put into practical use in the same manner as usual tablets. The term "a sufficient strength for handling relating to preparation" as used herein means a strength which can withstand at least the usual blister packaging, and such a strength will also withstand other handling such as delivery and carrying.

Hardness in the lengthwise direction of tablets may be used as an index of the strength which is applicable to blister packaging, namely a strength necessary to take out the tablet by pushing it out of a cover sheet of usual blister packaging. Such hardness varies depending on the size and shape of tablets and may preferably be 1.0 kg or more when the tablet has a diameter of about 8.0 mm, 1.5 kg or more for a diameter of about 10.0 mm and 2.0 kg or more for a diameter of about 12.0 mm. The solid preparation of the present invention has a sufficient strength for removal from blister packaging independent of its size.

As the strength required for bottle packaging (a a container made e.g. of glass or plastics in which tablets are placed), i.e., the strength required to withstand the contact between tablets and between the tablet and bottle wall when the bottle is transported, the tablet may preferably have a hardness of 3 kg or more. The preparation of the present invention has a sufficient strength for transport in bottles containing the preparation.

The term "quick disintegration and dissolution" as used herein means a practically sufficient disintegration or dissolution of the preparation by saliva in the buccal cavity without taking water. The term "practically sufficient disintegration or dissolution" means that the preparation disintegrates or dissolves in the buccal cavity within approximately 1 to 120 seconds, preferably within approximately 1 to 60 seconds, more preferably within approximately 1 to 40 seconds, though there are variations depending on the individual person.

The preparation of the present invention rapidly becomes brittle by saliva in the buccal cavity and gradually disintegrates or dissolves, and the disintegration or dissolution becomes quicker when an intrabuccal pressure, namely pressure between the upper jaw and tongue, or a "licking" movement, is applied to the preparation.

A person of parched mouth or having a small quantity of saliva in the buccal cavity may use the preparation of the present invention with the aid of cold or hot water in an amount sufficient to wet the buccal cavity.

Also, the preparation of the present invention may be swallowed together with a small amount of water after the preparation is disintegrated or dissolved in the buccal cavity or under a partly disintegrated or dissolved condition. Even by such a way of drug-taking, merits of the preparation of the present invention such as easy swallowing and small amount of water to be used can apply.

Of course, the preparation of the present invention can be taken together with water with no problems similar to the case of usual tablets. The preparation of the present invention can be used by any of these drug-taking means in accordance with each patient's choice or condition, provided that there are no limitations with respect to the active ingredient contained therein.

The following describes in detail, purely by way of illustration, embodiments of the process for the production of the intrabucally dissolving compressed moldings of the present invention.

### First process

An active ingredient is added to low moldability first saccharide and the resulting mixture is coated and/or granulated using high moldability second saccharide as a binder. In the resulting granules, granules of the active ingredient and granules of the low moldability saccharide are linked together with the high moldability saccharide. Preferably, the resulting granules may further be granulated with the high moldability saccharide as binder. The resulting granules are compression molded to obtain, for example, intrabuccally dissolving tablets.

### Second process

Low moldability first saccharide is coated and/or granulated using high moldability second saccharide as a binder. The granules are mixed with an active ingredient, and the mixture is compression molded to obtain, for example, intrabuccally dissolving tablets.

### Third process

Low moldability first saccharide is coated and/or granulated using high moldability second saccharide as a binder. Separately, an active ingredient is coated and/or granulated using high moldability second saccharide as a binder. These granules are mixed and compression molded to obtain, for example, intrabuccally dissolving tablets.

### Fourth process

Low moldability first saccharide is coated and/or granulated with both of an active ingredient and high moldability second saccharide binder in any order. The granules are compression molded to obtain, for example, intrabuccally dissolving tablets.

### Fifth process

Low moldability first saccharide (central core) is coated with high moldability second saccharide as a binder (first layer), then coated with an active ingredient (second layer); the resulting product is coated with high moldability second saccharide as a binder (third layer). The granules are compression molded to obtain, for example, intrabuccally dissolving tablets.

### Sixth process

Low moldability first saccharide is coated with an active ingredient and the coated product is coated and/or granulated using high moldability second saccharide as a binder. The granules are compression molded to obtain, for example, intrabuccally dissolving tablets.

The granulation may be carried out making use of, for example, a fluidized bed granulator (manufactured by Ohgawara Seisakusho), a vertical mixer (manufactured by San-ei Seisakusho) or an agitated granulating machine (manufactured by Fukae Kogyo), by mixing an active agent with low moldability first saccharide and other additive agents and coating and/or granu lating the resulting mixture using an aqueous solution of high moldability second saccharide as a binding agent. More illustratively, when a fluidized bed granulator is used, granulation is carried out to obtain granules having a desired particle size in accordance with the generally used operation conditions, for example, under a spray pressure of 0.3 to 2 kg/cm² and at a temperature of 20 to 30°C. In this instance, the effects of the present invention are further improved when fine granule coating is carried out as a pretreatment, before granulation, by means of a side spraying using a portion of the binding agent.

The compression molding may be carried out by tabletting using a tabletting machine generally used for the molding of tablets, such as a single tabletting machine (manufactured by Kikusui Seisakusho) or a rotary tabletting machine (manufactured by Hata Seisakusho) The molding pressure for tabletting may be selected depending on the hardness and dissolution property required of the resulting moldings.

The hardness of a tablet after tabletting can be further improved while maintaining the dissolution property by appropriately utilizing a step comprising spraying a physiologically acceptable organic solvent or water and drying, or a step comprising humidity treatment and drying.

The preparation of the present invention may contain various additive agents generally used in the production of tablets as long as they do not spoil the effects of the present invention.

Such additive agents include disintegrating agents, binding agents, souring agents, vesicants, artificial sweeteners, perfumes, lubricants and coloring agents.

Illustrative examples of disintegrating agents include starches such as corn starch and potato starch, as well as carmellose calcium. Illustrative examples of binding agents include powdered acacia, gelatin and pullulan.

Illustrative examples of souring agents include citric acid, tartaric acid and malic acid. Illustrative examples of vesicants include sodium bicarbonate. Illustrative examples of artificial sweeteners include saccharin sodium, glycyrrhizin dipotassium, aspartame, stevia and thaumatin.

Illustrative examples of perfumes include lemon, lemon lime, orange and menthol. Illustrative examples of lubricants include magnesium stearate, sucrose fatty acid ester, polyethylene glycol, talc and stearic acid. Illustrative examples of coloring agents include food dyes such as Food Yellow No. 5, Food Red No. 2 and Food Blue No.2, as well as food lake dyes and red ferric oxide.

These additive agents may be used alone or as a mixture of two or more in an appropriate amount at an optional step in the production process of the intrabuccally dissolving compressed moldings - for example, when an active ingredient is mixed with low moldability first saccharide, when a coating solution prepared by dissolving an active ingredient together with high moldability second saccharide in water is mixed, or before or after these steps.

The intrabuccally dissolving compressed moldings of the present invention show excellent dissolution inherent to low moldability saccharides and excellent disintegration resulting from the high dissolution, because each of the moldings uses low moldability first saccharide as its main component, with a blending ratio of high moldability second saccharide to low moldability first saccharide of from 2 to 20% by weight, preferably from 5 to 10% by weight, while the use of the high moldability second saccharide gives other useful physical properties such as adequate hardness, which cannot be found in conventional intrabuccally dissolving compressed moldings.

The intrabuccally dissolving compressed moldings of the present invention are produced through conventionally used production steps, namely granulation and tabletting, without employing a freeze drying step which is essential for the production of conventional intrabuccally dissolving compressed moldings. In consequence, the moldings of the present invention can be produced economically with high industrial productivity, because special facilities for freeze drying are not required.

In addition, the adequate hardness of the intrabuccally dissolving compressed moldings of the present invention renders possible easy handling of the moldings during their production steps and distribution stages.

The intrabuccally dissolving compressed molding of the present invention can be applied to the cases in which an active ingredient is absorbed in the buccal cavity as the occasion demands.

Hardness can be tested in the usual way, for example, by subjecting test samples to a Schleuniger tablet hardness meter (manufactured by Schleuniger). The following examples are provided to further illustrate the present invention.

### (Test Examples)

In order to describe the effects of the present invention further in detail, properties of the tablets obtained in Examples were measured in the following manner.

### (1) Hardness test

The hardness was measured using a tablet hardness meter (manufactured by Schleuniger). Each test was carried out 3 to 10 times (n = 3 to 10), and the average value was used in the following.

### (2) Disintegration dissolution test in the buccal cavity without water

A compressed molding sample was put into the buccal cavity of each of healthy male adult volunteers without water (not holding water in the mouth) to measure the time required for the complete disintegration and dissolution of the sample by saliva in the buccal cavity.

### (3) Disintegration test

Disintegration was measured in accordance with the disintegration test described in *The Japanese Pharmacopoeia,* 12th revision, (to be referred to as "JP disintegration test" hereinafter). Each test was carried out 6 times, and the average value was used in the following.

Since the physicochemical properties and the amount of the active ingredient influence the hardness and dissolution time in the buccal cavity of the moldings of the present invention only in rare cases, the active ingredient was not used in some of the following Examples.

### EXAMPLE 1

A 20 g portion of maltose (manufactured by Hayashibara Shoji) was dissolved in 180 g of water. Using the resulting aqueous solution of maltose, 400 g of mannitol (manufactured by Towa Kasei Kogyo) was subjected to granulation making use of a fluidized bed granulator (manufactured by Ohgawara Seisakusho). In this case, fine particle coating was carried out under a spray pressure of 3 kg/cm² for the first 10 g of maltose and then granulation was carried out under a spray pressure of 0.5 kg/cm² (mean particle diameter: 184 µm). After drying, magnesium stearate was blended in an amount of 0.5% and the resulting granules were applied to a rotary tabletting machine (manufactured by Hata Seisakusho) using a punch of R 10 mm and φ10 mm to obtain tablets each weighing 300 mg. The hardness test was repeated 3 times (n = 3).

### EXAMPLE 2

The procedure of Example 1 was repeated except that maltitol (manufactured by Towa Kasei Kogyo) was used instead of maltose. The mean particle diameter of the granules was 158 µm.

### EXAMPLE 3

The procedure of Example 1 was repeated except that sorbitol (manufactured by Towa Kasei Kogyo) was used instead of maltose. The mean particle diameter of the granules was 146 µm.

### EXAMPLE 4

The procedure of Example 1 was repeated except that lactose (manufactured by Domo Milk) was used instead of mannitol. The hardness test was repeated 3 times (n = 3). The mean particle diameter of the granules was 136 µm.

### EXAMPLE 5

The procedure of Example 1 was repeated except that an oligosaccharide (Nyuka Oligo LS-55P, manufactured by Hayashibara Shoji) was used instead of maltose. The hardness test was repeated 3 times (n = 3). The mean particle diameter of the granules was 192 µm.

### EXAMPLE 6

After mixing 200 g of lactose with 200 g of mannitol, granulation was carried out making use of a fluidized bed granulator using 20 g of maltitol dissolved in 80 g of water. In this case, granulation was carried out under a spray pressure of 0.5 kg/cm² (mean particle diameter: 202 µm). After drying, magnesium stearate was blended in an amount of 0.5% and the resulting granules were applied to a rotary tabletting machine using a punch of R 10 mm and φ10 mm to obtain tablets each weighing 300 mg. The hardness test was repeated 3 times (n = 3).

**TABLE 1**

| Example | Ratio of Low moldability sugar/ High moldability sugar | Tabletting pressure | Hardness | Time *¹ |
|---|---|---|---|---|
| | | (kg/punch) | (kg) | (sec) |
| 1 | mannitol:maltose | 303 | 5.9 | 15 |
| | = 20:1 | | | |
| | | | | |
| 4 | lactose:maltose | 334 | 5.3 | 15 |
| | = 20:1 | | | |
| | | | | |
| 5 | mannitol:oligosaccharide | 441 | 3.6 | 20 |
| | = 20:1 | | | |
| | | | | |
| 6 | mannitol:lactose:maltose | 388 | 3.7 | 16 |
| | = 10:10:1 | | | |

| | | | | |
|---|---|---|---|---|
| *¹: disintegration-dissolution time in the buccal cavity | | | | |

### EXAMPLE 7

Granules (mean particle diameter: 295 µm) were prepared by repeating the procedure of Example 1 except that glucose (manufactured by Nippon Shokuhin Kako) was used instead of mannitol. After drying, the granules were applied to an oil press machine using a punch of 10 mmR and φ10 mm under a pressure of 20 kg/cm² to obtain tablets each weighing 300 mg.

### EXAMPLE 8

The procedure of Example 7 was repeated except that xylitol (manufactured by Towa Kasei Kogyo) was used instead of glucose.

### EXAMPLE 9

The procedure of Example 7 was repeated except that sucrose (manufactured by Nisshin Seito) was used instead of glucose. The mean particle diameter of the granules was 355 µm.

### EXAMPLE 10

After mixing 2.832 kg of mannitol, 2.832 kg of lactose, 1.0 kg of famotidine, and 0.225 kg of aspartame, granulation was carried out, in which fine particle coating was carried out under a spray pressure of 4.0 kg/cm² for the first 1.0 kg of 15% maltose aqueous solution and then granulation was carried out. Then, 77.8 g of β-cyclodextrin and 8.6 g of menthol suspended in water was sprayed to the resulting granules in the same manner. After drying, calcium stearate was blended in an amount of 1% and the resulting granules were applied to a rotary tabletting machine using a punch of R 9.6 mm and φ8 mm under a pressure of 84 kg/punch to obtain tablets each weighing 150 mg. The resulting tablets showed a disintegration dissolution time of 15 seconds in the buccal cavity and a hardness (n = 5) of 3.9 kg.

### EXAMPLE 11

After mixing 20 g of famotidine, 270 g of lactose, 40 g of mannitol, 8 g of aspartame, and 2 g of sodium citrate, granulation was carried out making use of a fluidized bed granulator (manufactured by Ohgawara Seisakusho) using 16 g of maltose dissolved in 144 g of water. In this case, fine particle coating was carried out under a spray pressure of 3 kg/cm² for the first 8 g of maltose and then granulation was carried out under a spray pressure of 0.5 kg/cm² (mean particle diameter: 198 µm). After granulation, 0.34 g of menthol and 2.46 g of β-CD suspended in hot water was sprayed to the resulting granules in the same manner. After drying, magnesium stearate was blended in an amount of 0.5% and the resulting granules were applied to a rotary tabletting machine (manufactured by Hata Seisakusho) using a punch of R 10 mm and φ10 mm under a pressure of 133 kg/punch to obtain tablets each weighing 355.3 mg. The resulting tablets showed a disintegration dissolution time of 15 seconds in the buccal cavity and a hardness (n = 3) of 3.8 kg.

### EXAMPLE 12

A 21 g portion of maltose was dissolved in 189 g of water. Using the resulting aqueous solution of maltose, a mixture of 396.9 g of mannitol and 3.5 g of glibenclamide was subjected to granulation making use of a fluidized bed granulator. In this case, fine particle coating was carried out under a spray pressure of 3 kg/cm² for the first 8 g of maltose and then granulation was carried out under a spray pressure of 0.6 kg/cm² (mean particle diameter: 127 µm). After drying, magnesium stearate was blended in an amount of 0.5% and the resulting granules were applied to a rotary tabletting machine using a punch of R 10 mm and φ10 mm under a pressure of 319 kg/punch to obtain tablets each weighing 300 mg. The resulting tablets showed a disintegration dissolution time of 15 seconds in the buccal cavity and a hardness (n = 10) of 3.0 kg.

### EXAMPLE 13

Using 10 g of maltose dissolved in 90 g of water, 400 g of mannitol was subjected to granulation making use of a fluidized bed granulator (manufactured by Ohgawara Seisakusho) (mean particle diameter: 98 µm). In this case, fine particle coating was carried out under a spray pressure of 3 kg/cm². After drying, the resulting granules were applied to an oil press machine using a punch of R 10 mm and φ10 mm under a pressure of 50 kg/cm² to obtain tablets each weighing 300 mg. The resulting tablets showed a disintegration dissolution time of 15 seconds in the buccal cavity and a hardness (n = 3) of 4.8 kg.

### EXAMPLE 14

A 35 g portion of maltose was dissolved in 140 g of water. Using the resulting aqueous solution of maltose, 350 g of mannitol was subjected to granulation making use of a fluidized bed granulator (manufactured by Ohgawara Seisakusho). In this case, fine particle coating was carried out under a spray pressure of 3 kg/cm² for the first 16 g of maltose and then granulation was carried out under a spray pressure of 0.5 kg/cm² (mean particle diameter: 329 µm). After drying, magnesium stearate was blended in an amount of 0.5% and the resulting granules were applied to a rotary tabletting machine using a punch of R 10 mm and φ10 mm to obtain tablets each weighing 300 mg. The resulting tablets showed a disintegration dissolution time of 18 seconds in the buccal cavity and a hardness (n = 3) of 3.0 kg.

### EXAMPLE 15

Using aqueous solutions of maltose, a mixture of 4 kg of mannitol and 4 kg of lactose was subjected to granulation making use of a fluidized bed granulator (FLO-5, manufactured by Ohgawara Seisakusho). In this case, fine particle coating was carried out under a spray pressure of 3 kg/cm² using a 10% maltose aqueous solution for the first 0.2 kg of maltose and then granulation was carried out under a spray pressure of 1.5 kg/cm² using a 30% maltose aqueous solution for the next 0.4 kg of maltose (mean particle diameter: 140 µm). After drying, 240.4 g of the resulting granules were mixed with 8.3 g of famotidine and 1.25 g of magnesium stearate, and the resulting mixture was applied to a rotary tabletting machine using a punch of R 10 mm and φ10 mm to obtain tablets each weighing 300 mg. The resulting tablets showed a disintegration dissolution time of 20 seconds in the buccal cavity and a hardness (n = 5) of 3.6 kg.

### EXAMPLE 16

Using aqueous solutions of maltose, 8 kg of mannitol was subjected to granulation making use of a fluidized bed granulator (FLO-5, manufactured by Ohgawara Seisakusho). In this case, fine particle coating was carried out under a spray pressure of 2.5 kg/cm² using a 10% maltose aqueous solution for the first 2.0 kg of maltose and then granulation was carried out under a spray pressure of 1.5 kg/cm² using a 20% maltose aqueous solution for the next 0.4 kg of maltose.

Separately, 500 g of acetaminophen was subjected to granulation making use of a fluidized bed granulator (Unigrat, manufactured by Ohgawara Seisakusho) using 25 g of maltose as a 10% maltose aqueous solution.

A 63 g portion of the resulting acetaminophen granules (mean particle diameter: 120 µm) were mixed with 235.5 g of the previously prepared mannitol granules (mean particle diameter: 134 µm) and 1.5 g of magnesium stearate, and the resulting mixture was applied to a rotary tabletting machine using a punch of R 10 mm and φ10 mm to obtain tablets each weighing 300 mg. The resulting tablets showed a disintegration dissolution time of 20 seconds in the buccal cavity and a hardness (n = 5) of 4.1 kg.

### EXAMPLE 17

Using aqueous solutions of maltose, a mixture consisting of 487.5 g of mannitol and 162.5 g of lactose was subjected to granulation making use of a fluidized bed granulator (Unigrat, manufactured by Ohgawara Seisakusho). In this case, fine particle coating was carried out under a spray pressure of 3 kg/cm² using a 10% maltose aqueous solution for the first 13 g of maltose and then coating was carried out under the same conditions using a solution which had been prepared by dissolving 138 mg of YM934 (2-(3,4-dihydro-2,2-dimethyl-6-nitro-2H-1,4-benzoxazin-4-yl)pyridine N-oxide) in 50 ml of methanol. Thereafter, granulation was carried out under a spray pressure of 1.3 kg/cm² using 19.6 g of maltose as a 20% aqueous solution.

After drying, 628.1 g of the resulting granules (mean particle diameter: 161 µm) were mixed with 1.89 g of magnesium stearate, and the resulting mixture was applied to a rotary tabletting machine using a punch of R 10 mm and φ10 mm to obtain tablets each weighing 294 mg. The resulting tablets showed a disintegration time of 25 seconds by the JP disintegration test and a hardness (n = 10) of 4.5 kg.

### EXAMPLE 18

Using 2.67 kg of a 15% maltose aqueous solution, 8 kg of mannitol was subjected to granulation making use of a fluidized bed granulator (FLO-5, manufactured by Ohgawara Seisakusho), and dried. In this case, fine particle coating was carried out under a spray pressure of 3.0 kg/cm² for the first 1.0 kg of the maltose aqueous solution and then granulation was carried out. Separately, 500 g of calcium carbonate was suspended in a solution which had been prepared by dissolving 50 g of maltose in 367 g of water. The resulting suspension was spray dried using a spray drying machine manufactured by Ohgawara Kakoki. A 110 g portion of the resulting spray-dried product, 132 g of the previously prepared mannitol granules, 20 g of magnesium hydroxide and 1.2 g of magnesium stearate were mixed, and the resulting mixture was applied to a rotary tabletting machine using a punch of R 11 mm and φ11 mm under a pressure of 154 kg/punch to obtain tablets each weighing 525 mg. The resulting tablets showed a dissolution time of 25 seconds in the buccal cavity and a hardness (n = 5) of 3.7 kg.

### EXAMPLE 19

A 10 mg portion of salmon calcitonin, 100 mg of gelatin, and 890 mg of mannitol were mixed in a mortar to prepare a trituration-powder having 1% salmon calcitonin. This powder was mixed with 8 g of the mannitol granules prepared in Example 18, and the resulting mixture was applied to an oil press machine using a punch of R 9.6 mm and φ8 mm under a pressure of 20 kg/cm² to obtain tablets each weighing 112.5 mg (corresponding to 500 IU salmon calcitonin). The resulting tablets showed a dissolution time of 10 seconds in the buccal cavity and a hardness (n = 5) of 5.9 kg.

## Claims

1. An intrabuccally dissolving compressed molding which shows disintegration and dissolution in the buccal cavity within 1 to 120 seconds and which comprises (a) granules comprising first saccharide coated and/or granulated with second saccharide as a binder, and (b) optionally an active ingredient; said first saccharide being at least one saccharide selected from lactose, mannitol, glucose, sucrose, and xylitol and showing a hardness of 0 to 2 kg when 150 mg of the saccharide is tabletted using a punch of 8 mm diameter under a pressure of 10 to 50 kg/cm², and said second saccharide being at least one saccharide selected from maltose, maltitol, sorbitol and lactosucrose and showing a hardness of 2 kg or more when 150 mg of the saccharide is tabletted using a punch of 8 mm diameter under a pressure of 10 to 50 kg/cm².

2. A molding according to claim 1 wherein the blending ratio of said second saccharide to said first saccharide is 2 to 20% by weight.

3. A molding according to claim 2 wherein said ratio is 5 to 10% by weight.

4. A molding according to any preceding claim which is a tablet.

5. A process for producing a molding according to claim 1 or 2 or 3 which comprises coating and/or granulating said first saccharide using said second saccharide as a binder, and subjecting the resulting granules to compression molding.

6. A process according to claim 5 wherein said molding includes an active ingredient and the process comprises [a] coating and/or granulating an active ingredient and said first saccharide using said second saccharide as a binder, and subjecting the resulting granules to compression molding; or [b] coating and/or granulating said first saccharide using said second saccharide as a binder, mixing the resulting granules with an active ingredient, and subjecting the resulting mixture to compression molding; or [c] coating and/or granulating said first saccharide using a said second saccharide as a binder to obtain first granules, coating and/or granulating an active ingredient using a said second saccharide as a binder to obtain second granules, mixing the first and second granules, and subjecting the resulting mixture to compression molding; or [d] coating and/or granulating said first saccharide with both an active ingredient and said second saccharide binder in any order, and subjecting the resulting granules to compression molding; or [e] coating said first saccharide (central core) with a said second saccharide as a binder (first layer), coating the resulting product with an active ingredient (second layer), and coating the resulting product with a said second saccharide as a binder (third layer), thereby obtaining a three layer structure coat; or (f) coating said first saccharide with an active ingredient and coating and/or granulating the coated product using said second saccharide as a binder, and subjecting the resulting granules to compression molding.

7. A process according to claim 5 or 6 which includes adding at least one additive agent selected from a disintegrating agent, a binding agent, a souring agent, a vesicant, an artificial sweetener, a perfume, a lubricant and a coloring agent.

8. A process according to any of claims 5 to 7 wherein said compression molding is tabletting.

9. A process according to claim 8 which includes subjecting the resulting tablet to a humidity treatment followed by drying.

10. A process according to claim 8 which includes spraying a physiologically acceptable organic solvent or water onto the resulting tablet followed by drying.

## Patentansprüche

1. Intrabukkal auflösendes komprimiertes Formteil, das in der Wangenhöhle innerhalb von 1 bis 120 Sekunden zerfällt und sich auflöst und Folgendes umfasst: (a) Körnchen, die ein erstes Saccharid umfassen, das mit einem zweiten Saccharid als Bindemittel beschichtet und/oder granuliert ist, und (b) bei Bedarf einen Wirkstoff, wobei das genannte erste Saccharid wenigstens ein Saccharid ist, das ausgewählt ist aus Lactose, Mannitol, Glukose, Saccharose und Xylitol und eine Härte zwischen 0 und 2 kg aufweist, wenn 150 mg des Saccharids mit einer Stanze mit einem Durchmesser von 8 mm unter einem Druck von 10 bis 50 kg/cm² tablettiert werden, und wobei das genannte zweite Saccharid wenigstens ein Saccharid ist, das ausgewählt ist aus Maltose, Maltitol, Sorbitol und Lactosaccharose und eine Härte von 2 kg oder mehr aufweist, wenn 150 mg des Saccharids mit einer Stanze mit einem Durchmesser von 8 mm unter einem Druck von 10 bis 50 kg/cm² tablettiert werden.

2. Formteil nach Anspruch 1, wobei das Mischungsverhältnis zwischen dem genannten zweiten Saccharid und dem genannten ersten Saccharid zwischen 2 und 20 Gew.-% liegt.

3. Formteil nach Anspruch 2, wobei das genannte Verhältnis zwischen 5 und 10 Gew.-% liegt.

4. Formteil nach einem der vorherigen Ansprüche, das eine Tablette ist.

5. Verfahren zur Herstellung eines Formteils nach Anspruch 1 oder 2 oder 3, umfassend das Beschichten und/oder Granulieren des genannten ersten Saccharids mit dem genannten zweiten Saccharid als Bindemittel und Formpressen der resultierenden Körnchen.

6. Verfahren nach Anspruch 5, wobei das genannte Formteil einen Wirkstoff enthält und das Verfahren die folgenden Schritte umfasst: [a] Beschichten und/oder Granulieren eines Wirkstoffs und des genannten ersten Saccharids mit dem genannten zweiten Saccharid als Bindemittel und Formpressen der resultierenden Körnchen; oder [b] Beschichten und/oder Granulieren des genannten ersten Saccharids mit dem genannten zweiten Saccharid als Bindemittel, Vermischen der resultierenden Körnchen mit einem Wirkstoff und Formpressen des resultierenden Gemischs; oder [c] Beschichten und/oder Granulieren des genannten ersten Saccharids mit dem genannten zweiten Saccharid als Bindemittel, um erste Körnchen zu erhalten, Beschichten und/oder Granulieren eines Wirkstoffs mit dem genannten zweiten Saccharid als Bindemittel, um zweite Körnchen zu erhalten, Vermischen der ersten und der zweiten Körnchen und Formpressen des resultierenden Gemischs; oder [d] Beschichten und/oder Granulieren des genannten ersten Saccharids mit sowohl einem Wirkstoff als auch dem genannten zweiten Saccharidbindemittel in einer beliebigen Reihenfolge und Formpressen der resultierenden Körnchen; oder [e] Beschichten des genannten ersten Saccharids (zentraler Kern) mit dem genannten zweiten Saccharid als Bindemittel (erste Lage), Beschichten des resultierenden Produkts mit einem Wirkstoff (zweite Lage) und Beschichten des resultierenden Produkts mit dem genannten zweiten Saccharid als Bindemittel (dritte Lage), so dass eine dreilagige Strukturschicht entsteht; oder (f) Beschichten des genannten ersten Saccharids mit einem Wirkstoff und Beschichten und/oder Granulieren des beschichteten Produkts mit dem genannten zweiten Saccharid als Bindemittel und Formpressen der resultierenden Körnchen.

7. Verfahren nach Anspruch 5 oder 6, umfassend die Zugabe von wenigstens einem Zusatzstoff, ausgewählt aus einem Zerfallsmittel, einem Bindemittel, einem Säuerungsmittel, einem blasenziehenden Mittel, einem künstlichen Süßungsmittel, einem Parfüm, einem Schmiermittel und einem Färbemittel.

8. Verfahren nach einem der Ansprüche 5 bis 7, wobei das genannte Formpressen Tablettieren ist.

9. Verfahren nach Anspruch 8, umfassend das Unterziehen der resultierenden Tablette einer Feuchtigkeitsbehandlung, gefolgt von Trocknen.

10. Verfahren nach Anspruch 8, umfassend das Sprühen eines physiologisch akzeptablen organischen Lösungsmittels oder von Wasser auf die resultierende Tablette, gefolgt von Trocknen.

## Revendications

1. Pièce moulée comprimée se dissolvant intrabuccalement présentant désagrégation et dissolution dans la cavité bucale en l'espace de 1 à 120 secondes et qui comprend (a) des granules comprenant un premier saccharide enrobé et/ou granulé avec un deuxième saccharide en tant que liant, et (b) optionnellement un principe actif, ledit premier saccharide étant au moins un saccharide sélectionné parmi le lactose, le mannitol, le glucose, le saccharose et le xylitol et présentant une dureté de 0 à 2 kg lorsque 150 mg du saccharide sont mis en comprimés en utilisant un poinçon de 8 mm de diamètre sous une pression de 10 à 50 kg/cm², et ledit deuxième saccharide étant au moins un saccharide sélectionné parmi le maltose, le maltitol, le sorbitol et le lactosucrose et présentant une dureté de 2 kg ou plus lorsque 150 mg du saccharide sont mis en comprimés en utilisant un poinçon de 8 mm de diamètre sous une pression de 10 à 50 kg/cm².

2. Pièce moulée selon la revendication 1, où le rapport de mélange dudit deuxième saccharide audit premier saccharide est de 2 à 20% en poids.

3. Pièce moulée selon la revendication 2, où ledit rapport est de 5 à 10% en poids.

4. Pièce moulée selon l'une quelconque des revendications précédentes qui est un comprimé.

5. Procédé de production d'une pièce moulée selon la revendication 1, 2 ou 3, qui comprend enrober et/ou granuler ledit premier saccharide en utilisant ledit deuxième saccharide en tant que liant, et soumettre les granules résultants au moulage par compression.

6. Procédé selon la revendication 5, où ladite pièce moulée comprend un principe actif et le procédé comprend [a] enrober et/ou granuler un principe actif et ledit premier saccharide en utilisant ledit deuxième saccharide en tant que liant, et soumettre les granules résultants au moulage par compression; ou [b] enrober et/ou granuler ledit premier saccharide en utilisant ledit deuxième saccharide en tant que liant, mélanger les granules résultants avec un principe actif, et soumettre le mélange résultant au moulage par compression; ou [c] enrober et/ou granuler ledit premier saccharide en utilisant ledit deuxième saccharide en tant que liant pour obtenir des premiers granules, enrober et/ou granuler un ingrédient actif en utilisant ledit deuxième saccharide en tant que liant pour obtenir des deuxièmes granules, mélanger les premiers et deuxièmes granules, et soumettre le mélange résultant au moulage par compression; ou [d] enrober et/ou granuler ledit premier saccharide avec un principe actif et avec ledit deuxième saccharide liant dans n'importe quel ordre, et soumettre les granules résultants au moulage par compression; ou [e] enrober ledit premier saccharide (noyau central) avec un dit deuxième saccharide en tant que liant (première couche), enrober le produit résultant avec un principe actif (deuxième couche), et enrober le produit résultant avec un dit deuxième saccharide en tant que liant (troisième couche), obtenant ainsi un enrobage structuré en trois couches; ou [f] enrober ledit premier saccharide avec un principe actif et enrober et/ou granuler le produit enrobé en utilisant ledit deuxième saccharide en tant que liant, et soumettre les granules résultants au moulage par compression.

7. Procédé selon la revendication 5 ou 6, qui comprend ajouter au moins un agent additif sélectionné parmi un agent de désagrégation, un agent liant, un agent acidifiant, un vésicant, un édulcorant artificiel, un parfum, un lubrifiant et un agent colorant.

8. Procédé selon l'une quelconque des revendications 5 à 7, où ledit moulage par compression est la mise en comprimés.

9. Procédé selon la revendication 8, qui englobe soumettre le comprimé résultant à un traitement d'humidité suivi par le séchage.

10. Procédé selon la revendication 8, qui englobe pulvériser un solvant organique physiologiquement acceptable ou de l'eau sur le comprimé résultant suivi par le séchage.
